# EUROPEAN PATENT SPECIFICATION

(11) **EP 1 066 053 B1**
(45) Date of publication and mention of the grant of the patent: **09.08.2006**
(21) Application number: 99915692.0
(22) Date of filing: 22.03.1999
(51) Int. Cl.: A61K 39/295, A61K 39/39, A61K 39/05, A61K 39/08, A61K 39/09, A61K 39/095, A61K 39/10, A61K 39/102, A61K 39/13, A61K 39/29

(54) **HIB/DTPA VACCINE COMPOSITION AND METHODS OF PREPARATION**
HIB/DTPA IMPFSTOFF UND HERSTELLUNGSMETHODEN
COMPOSITION DE VACCIN CONTRE HIB/DTPA ET SES METHODES DE PREPARATION

(30) Priority: 25.03.1998 GB 9806456
(43) Date of publication of application: 10.01.2001
(73) Proprietor: Glaxosmithkline Biologicals S.A., 1330 Rixensart (BE)
(72) Inventor: ARTOIS, Claude, Glaxosmithkline Biologicals S.A., B-1330 Rixensart (BE); DE HEYDER, Koen, Glaxosmithkline Biologicals S.A., B-1330 Rixensart (BE); DESMONS, Pierre, Glaxosmithkline Biologicals S.A., B-1330 Rixensart (BE); GARCON, Nathalie, Glaxosmithkline Biologicals S.A., B-1330 Rixensart (BE); MAINIL, Roland, Glaxosmithkline Biologicals S.A., B-1330 Rixensart (BE)
(74) Representative: Dalton, Marcus Jonathan William
(86) International application number: PCT/EP1999/001959
(87) International publication number: WO 1999/048525

(56) References cited:
- WO-A-93/24148
- WO-A-97/00697
- WO-A-97/46255
- CORBEL M.J. ET AL: "Workshop on Standardisation of Aluminium Adsorbed Vaccines, Bergen, Norway, 21 June 1996." BIOLOGICALS, (1997) 25/3 (351-353). , XP002110484
- CORBEL M J: "CONTROL TESTING OF COMBINED VACCINES: A CONSIDERATION OF POTENTIAL PROBLEMS AND APPROACHES" BIOLOGICALS, vol. 22, no. 4, 1 December 1994 (1994-12-01), pages 353-360, XP000603003 ISSN: 1045-1056
- ELLIS R W: "Development of combination vaccines" VACCINE, vol. 17, no. 13-14, January 1999 (1999-01), page 1635-1642 XP004158298 ISSN: 0264-410X

## Description

The present invention relates to a method to reduce interference of the capsular polysaccharide component of a conjugated *Haemophilus influenzae* B vaccine (Hib) in a combination vaccine comprising DTPa - a well known 'trivalent' combination vaccine comprising Diphtheria toxoid (DT), tetanus toxoid (TT), and acellular *Pertussis* components [typically detoxified *Pertussis* toxoid (PT) and filamentous haemagglutinin (FHA) with optional pertactin (PRN) and/or agglutinin 1+2], for example the marketed vaccine INFANRIX-DTPa^{™} (SmithKline Beecham Biologicals) which contains DT, TT, PT, FHA, and PRN antigens. The invention also relates to combination vaccines that are made by this method.

Vaccines that utilise polysaccharides are known in the art. For example a Hib vaccine for the prevention of *Haemophilus influenzae* B infections is based on the *H. influenzae* B capsular polysaccharide (PRP) conjugated with a carrier protein. The polysaccharide is a polymer of ribose, ribitol and phosphate. Examples of carrier protein include diphtheria or tetanus toxoid, or an outer membrane protein of *N. meningitidis.* See for example US 4,365,170, US 4,673,574, EP 208375, EP 477508 and EP 161188.

It is desirable to administer such conjugate vaccines with other antigens or vaccines at the same time and this can involve multiple injections. Problems associated with multiple injections include a more complicated administration procedure and a large total injection volume. This is a particularly acute problem when the vaccine is intended for infants. For both the infant and the practitioner it is desirable to inject all necessary antigens in one shot of normal volume, thus rendering the vaccination procedure less traumatic and painful for the infant, and more efficient and easier to manage for the practitioner.

It has therefore been proposed to combine such polysaccharide conjugate vaccines with other vaccines such as DTPa to produce more elaborate combination vaccines. In addition, the inclusion of further antigens to such a combination vaccine for the prevention of diseases like hepatitis B or Polio has also been proposed (combination vaccines comprising an antigen against hepatitis B and antigens against diphtheria, tetanus and pertussis (HepB, DTPa) have been described in WO 93/24148 and WO 97/46255).

It has been found, however, that simple mixing of the components of a combination vaccine is complicated by the fact that not all antigens can be effectively mixed together. The reduction in the immunogenicity of an antigen when combined with other components (as compared to the particular antigen administered alone) is known as interference. It is known, for example, that the extemporaneous mixing of a DTPa combination vaccine with unadjuvanted Hib results in a reduction of antibody titres to the polysaccharide component of Hib (WO 97/00697). In addition, WO 97/00697 showed that if Hib is adsorbed onto aluminium hydroxide, there is a significant reduction of antibody titres to the polysaccharide component. These results indicated that there was interference between the aluminium hydroxide of the DTPa vaccine and Hib. In order to try and minimise this interference in such an extemporaneously-prepared combination vaccine Hib was pre-adsorbed onto aluminium phosphate.

WO 96/37222 also describes this problem. In this case the antigenicity of Hib is stabilised by adsorbing it and the other DTPa components onto an aluminium-based adjuvant with a zero point charge of less than 7.2, for instance aluminium phosphate, or aluminium hydroxide to which anions have been added. However, when a uminium phosphate is used the approach can result in the increase in desorption of other antigens in the combination vaccine that work better when adsorbed onto aluminium hydroxide adjuvant, which can also be a problem when aluminium hydroxide plus anions is used (the surface of the aluminium hydroxide having been essentially coated by the anion).

A further problem encountered in the formulation of both monovalent and combination vaccines is the inherent stability of their composite antigens over time. Vaccines in solution may undergo processes over time which decrease the immunogenicity of its antigen components, for instance the degradation of the antigen or the desorption of the antigen from the adjuvant to which it had been adsorbed. Although generally it is desirable to aim for the highest possible degree of adsorption cf antigen onto adjuvant, for a reliable vaccine the main aim is to achieve the highest level of adsorption that can be maintained throughout the shelf-life of the vaccine. Even though lyophilised vaccines can be solubilised extemporaneously, it is advantageous if the solubilised vaccine is stable so that it will be effective over a long time-frame in order to give the practitioner the greatest flexibility with the minimum waste of vaccine. Ideally, a stable vaccine could be provided in solution (a "liquid" vaccine). A stable vaccine in solution would not only be less costly to manufacture than a lyophilised equivalent, it would also remove an additional handling step by the practitioner which may be at risk of being carried out incorrectly.

### BRIEF DESCRIPTION OF THE DRAWINGS

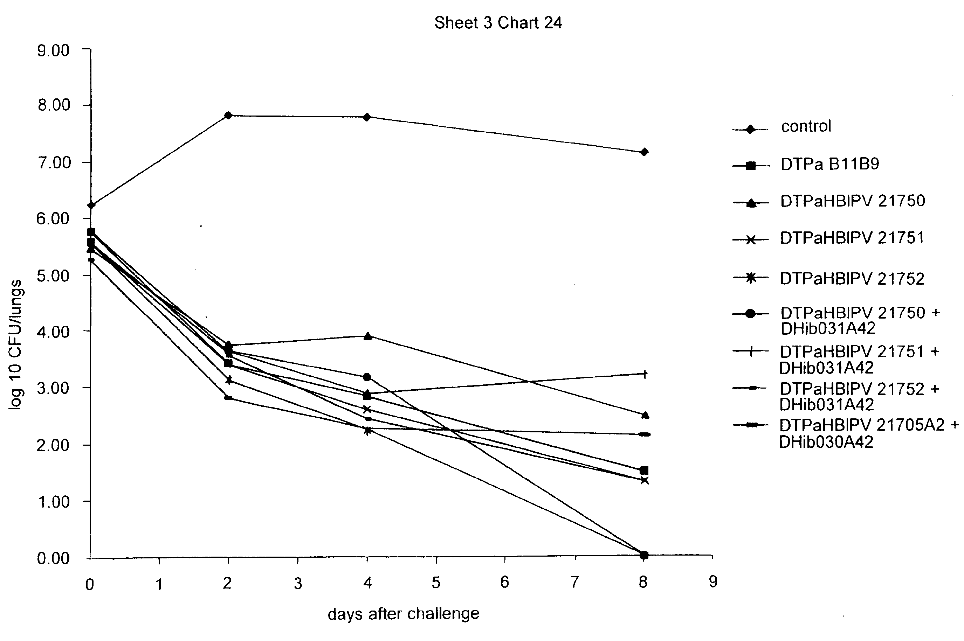

Figure 1: Graph showing the pertussis mouse lung clearance activity of 'Light Aluminium Hydroxide' (lot 21750 and 21751) and 'All Aluminium Phosphate' (lot 21752) formulations (with and without Hib) compared with a standard commercially available DTPa vaccine (indicated with square symbols).

### DESCRIPTION OF THE INVENTION

This invention relates to a general method by which either extemporaneously-prepared or liquid Hib/DTPa combination vaccines can be made in order to avoid better the Hib interference problem over that which has been described in the prior art whilst being able to maintain the maximum, stable adsorption of each antigen onto the aluminium-based adjuvant on which it is most immunogenic. In so doing, pertussis antigens in combination vaccines of the present invention are stably retained in their most potent form. In addition, the invention relates to how the method is compatible with an additional method to improve the adsorption of the components of a liquid combination vaccine onto aluminium-based adjuvants in a stable manner. In this way, the immunogenicity of the polysaccharide component of Hib in combination vaccines can be consistently maintained, and can remain stable in the liquid state for a greater period of time. Consequently, vaccines so produced are of greater use when administered to subjects, particularly children.

Specifically, this invention teaches a reliable method whereby aluminium hydroxide may be used as an adjuvant in a DTPa-Hib combination vaccine without causing interference to the Hib component over time in extemporaneously-prepared or liquid formulations, and without gratuitously adding anions to the formulation as does the method disclosed in WO 96/37222. As anions are not gratuitously added to the formulation, the stability of all the adsorbed antigens is maximised, and furthermore the method is compatible with a further disclosed method of optimising the adsorption of antigens onto aluminium-based adjuvants in a combination vaccine in a stable manner, specifically by optimising their adsorption (and minimising subsequent desorption) onto adjuvant over time in a liquid formulation, by using "extra-washed" aluminium phosphate.

Accordingly the present invention provides a method to reduce interference of a capsular polysaccharide component of a conjugated *Haemophilus influenzae* B vaccine (Hib) in a combination vaccine comprising DTPa, wherein such method comprises:
(i) selecting one or more antigen(s) to be adsorbed onto aluminium hydroxide adjuvant;
(ii) pre-saturating the aluminium hydroxide adjuvant with the selected antigens such that there is a minimisation of any interference to Hib antigen in the combination vaccine caused by aluminium hydroxide adjuvant;
(iii) selecting Hib plus one or more additional antigen(s) to be adsorbed onto aluminium phosphate;
(iv) adsorbing Hib and said additional antigens onto aluminium phosphate;
(v) combining all antigens in said vaccine.

By 'pre-saturating' is meant that antigen(s) that require aluminium hydroxide adjuvanting are pre-adsorbed onto aluminium hydroxide such that there is no excess aluminium hydroxide present sufficient to cause significant interference to Hib (whether unadsorbed or adsorbed onto aluminium phosphate) in the final combination vaccine. and that time is given in order for adsorption to take place before the aluminium hydroxide adsorbed antigens are combined with other antigens of the vaccine. Preferably the adsorption onto aluminium hydroxide should be left to reach completion (for at least a period of about 10 minutes at room temperature). The pre-saturation of the aluminium hydroxide thus results in a minimisation of any interference to the Hib antigen caused by aluminium hydroxide adjuvant. Often, the flocculation (or aggregation) state of the aluminium hydroxide adjuvant in the combination vaccine may be an indicator of Hib interference - the greater the flocculation, the greater the chance of Hib interference. In addition, using antigens to pre-saturate the aluminium hydroxide has the further advantage of incorporating less aluminium hydroxide per fixed dose of antigen which can potentially reduce the local reactogenicity associated with alum.

It should be appreciated that although the above method is most preferred, the method may also be carried out where the Hib component is not adjuvanted when added to the formulation.

Another aspect provided by this invention is a method whereby either unadjuvanted Hib or Hib adsorbed onto aluminium phosphate can be extemporaneously and effectively combined with the combination vaccine with the minimum decrease in its immunogenicity. Such a method comprises the following steps:
(i) selecting one or more antigen(s) to be adsorbed onto aluminium hydroxide adjuvant;
(ii) pre-saturating the aluminium hydroxide adjuvant with the selected antigens such that there is a minimisation of any interference to Hib antigen in the combination vaccine caused by aluminium hydroxide adjuvant;
(iii) selecting one or more additional antigen(s) to be adsorbed onto aluminium phosphate;
(iv) adsorbing said additional antigens onto aluminium phosphate;
(v) combining all antigens in said vaccine;
(vi) extemporaneously adding either unadjuvanted Hib or Hib adsorbed onto aluminium phosphate.

By 'DTPa' is meant the definition given above. DT, TT, PT, FHA and PRN are well known in the art. The PT component may be made into a toxoid either chemically or genetically, for example as described in EP 515415. See also EP 427462 and WO 91/12020 for the preparation of pertussis antigens. Optionally the PT component may be recombinant (for example as described in European Patent Applications EP 306318, EP 322533, EP 396964, EP 322115 and EP 275689). Optionally the DT and TT components may also be recombinant. Typically the PT, FHA, PRN, HBsAg (Hepatitis B surface antigen), and Hib components will be in the range 8-25 µg per 0.5 mL dose of bulk vaccine. The DT. TT, and IPV (inactivated trivalent poliovirus vaccine) components should typically be present as approximately 15-25 Lf (flocculating units), 10 Lf, and 40/8/32 (type I/II/III) DU respectively per 0.5 mL dose of bulk vaccine.

Preferably the carrier protein used in the Hib conjugate is either diphtheria toxoid, tetanus toxoid, Diphtheria Crm₁₉₇ protein or an outer membrane protein from a bacteria such as *N. meningitidis.* The synthesis of *Haemophilus influenzae* type B capsular polysaccharide (PRP) tetanus toxoid (TT) conjugate is described in WO 97/00697.

The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heteroligation chemistry involving the formation of the thioether linkage. Such conjugates are described in PCT published application WO93/15760 (Uniformed Services University).

The conjugates can also be prepared by direct reductive amination methods as described in US 4365170 (Jennings) and US 4673574 (Anderson). Other methods are described in EP 161188, EP 208375 and EP 477508.

A further method involves the coupling of a cyanogen bromide activated polysaccharide derivatised with adipic acid hydrazide (ADH) to the protein carrier by carbodiimide condensation. Such conjugation is described in Chu C. *et al*. Infec. Immunity, 1983 **245** 256.

In addition to DTPa antigens and Hib, the combination vaccine may also comprise other antigens, preferably one or more from the group: Hepatitis B surface antigen (HBsAg), inactivated Hepatitis A virus, Polio virus antigens (for instance inactivated trivalent polio virus - IPV), *N. meningitidis* A capsular polysaccharide (and protein conjugates thereof), *N. meningitidis* C capsular polysaccharide (and protein conjugates thereof), *Streptococcus pneumoniae* capsular polysaccharide (and protein conjugates thereof), *Streptococcus pneumoniae* proteins. *Moraxella catarrhalis* outer membrane proteins, non-typeable *Haemophilus influenzae* outer membrane proteins, *N. meningitidis* B outer membrane proteins.

Preferably, the HBsAg will be adsorbed onto aluminium phosphate as described in WO 93/24148. In addition, methods of adsorbing DTPa and Hib antigens onto aluminium adjuvants are known in the art. See for example WO 93/24148 and WO 97/00697.

Suitable components for use in such vaccines are already commercially available and details may be obtained from the World Health Organisation. For example the IPV component may be the Salk inactivated polio vaccine. The Hepatitis B surface antigen may comprise the 'S' antigen as in Engerix-B^{™} (SmithKline Beecham Biologicals). The inactivated Hepatitis A virus may preferably comprise the commercial vaccine known as Havrix^{™} (SmithKline Beecham Biologicals) which is a killed attenuated vaccine derived from the HM-175 strain of the virus.

The addition of either lyophilised or liquid Hib (either unadjuvanted or adsorbed onto aluminium phosphate) to a solution of the other components of the combination vaccine may be performed extemporaneously, or before the vaccine leaves the manufacturer. In either of the above cases the immunogenicity of the Hib component should remain stable and effective with minimal interference. Hib may be added either on its own or in a mixture with other components not adjuvanted with aluminium hydroxide.

Vaccine preparation is generally described in Vaccine Design - The Subunit and adjuvant approach Ed Powell and Newman; Pellum Press. Advantageously the combination vaccine according to the invention is a paediatric vaccine.

The amount of conjugate antigen in each vaccine dose is selected as an amount which induces an immunoprotective response without significant, adverse side effects in typical vaccinees. Such amount will vary depending on which specific immunogens are employed. Generally it is expected that each dose will comprise 1-1000 µg of conjugated polysaccharide (expressed in amount of polysaccharide), preferably 2-100 µg, and most preferably 4-40 µg. An optimal amount for a particular vaccine can be ascertained by standard studies involving observation of antibody titres and other responses in subjects. Following an initial vaccination, subjects may receive one or two booster injections at about 4 weeks intervals or longer.

In a preferred embodiment of the invention the ratio of aluminium phosphate to aluminium hydroxide adjuvant (expressed as relative amounts of Al³⁺) present in the combination vaccine ranges preferably from 1:1 to 20:1, more preferably from 2:1 to 14:1, and most preferably from 1.5:1 to 14:1. It is preferable to adsorb the PRN component onto aluminium hydroxide in the formulation as the inventors have found that whilst the antigen is fully adsorbed onto aluminium hydroxide over a wide range of pH values, when it is adsorbed onto aluminium phosphate it rapidly becomes poorly adsorbed (and is less potent) at pHs greater than 5.1. After 24 hours adsorption at pH 6.1 there is approximately less than 10% unadsorbed PRN using aluminium hydroxide versus approximately more than 50% using aluminium phosphate. As the final pH specified by WHO for DTP vaccines is between 6 and 7, it is therefore important to adsorb PRN onto aluminium hydroxide for the greatest efficacy. PRN has been shown to be one of the most important components in the pertussis vaccine, and it has now been discovered that a minimisation of adsorption reduces the T-cell response and the potency of the pertussis vaccine as a whole.

In another preferred embodiment of the invention all the antigens of the combination vaccine are adsorbed onto aluminium phosphate, with the proviso that pertactin is adsorbed onto aluminium hydroxide. This combination vaccine uses what is called the 'All Aluminium Phosphate' approach. Preferably, the pertactin adsorbed onto aluminium hydroxide is combined with unadsorbed inactivated Polio virus antigens before being combined with the aluminium phosphate adsorbed antigens (thus further ensuring that the aluminium hydroxide is pre-saturated). An example of this is described in Example 1.

The 'All Aluminium Phosphate' approach may also be used for the alternative method detailed above which adds Hib extemporaneously either in an unadjuvanted form, or adsorbed onto aluminium phosphate.

In a further preferred embodiment of the invention all the antigens of the combination vaccine are adsorbed onto aluminium hydroxide, with the proviso that HBsAg and Hib are adsorbed onto aluminium phosphate. This combination vaccine uses what is called the 'Light Aluminium Hydroxide' approach. Preferably, the antigens adsorbed onto aluminium hydroxide are combined before the HBsAg, adsorbed onto aluminium phosphate, is added. Preferably the HBsAg is combined with the other antigens prior to addition of Hib adsorbed onto aluminium phosphate. An example of this is described in Example 2. If, however, the total amount of adjuvant (aluminium salts) used is not enough to generate an adjuvant effect, free aluminium phosphate can be added at this time without negating the advantages of this invention.

The 'Light Aluminium Hydroxide' approach may also be used for the alternative method detailed above which adds Hib extemporaneously either in an unadjuvanted form or adsorbed onto aluminium phosphate. In such case any additional free aluminium phosphate that is added to the combination vaccine should be preferably done prior to the addition of Hib.

'Hybrid All Aluminium Phosphate / Light Aluminium Hydroxide' is yet a further preferred embodiment of the invention. In such a hybrid formulation selected antigens in addition to pertactin are adsorbed onto aluminium hydroxide, but not as many as in 'Light Aluminium Hydroxide' formulations. Again, all the aluminium hydroxide used is pre-saturated with antigen so as to minimise the risk of Hib interference. Preferably, all the antigens of the combination vaccine are adsorbed onto aluminium phosphate, with the proviso that pertactin, diphtheria toxoid, pertussis toxoid, filamentous haemagglutinin are adsorbed onto aluminium hydroxide. Again Hib may be either unadjuvanted or adsorbed onto aluminium phosphate when added to the liquid or extemporaneously-prepared formulations. Preferably, the pertactin, diphtheria toxoid, pertussis toxoid, filamentous haemagglutinin adsorbed onto aluminium hydroxide are combined with unadsorbed inactivated Polio virus antigens before being combined with the aluminium phosphate adsorbed antigens. An example of this is described in Example 3.

It should additionally be appreciated that one or more of the antigens selected to presaturate the aluminium hydroxide adjuvant may already be adsorbed onto another adjuvant, preferably aluminium phosphate. Such formulations are named 'Adapted All Aluminium Phosphate / Light Aluminium Hydroxide' formulations, and these provide a still further preferred embodiment of the invention. Again, all the aluminium hydroxide used is pre-saturated with antigen so as to minimise the risk of Hib interference. In such formulations Hib should be added to the formulation extemporaneously either in an unadjuvanted state, or adsorbed onto aluminium phosphate. Preferably, all the antigens of the combination vaccine are adsorbed onto aluminium phosphate, with the proviso that pertactin, pertussis toxoid, filamentous haemagglutinin are adsorbed onto aluminium hydroxide, and diphtheria toxoid (which has previously been adsorbed onto aluminium phosphate) is also combined with free aluminium hydroxide before combining it with the other antigens. An example of this is described in Example 4.

Preferably, the aluminium phosphate used in performing the method of the invention is 'extra-washed' prior to the adsorption of antigen such that the free phosphate ion concentration is reduced to below 10 mM and preferably 3 mM or less, or more preferably 2.5 mM or less. The extra-washed aluminium phosphate should be sterilised (preferably by autoclaving) before adsorption of antigen. Using extra-washed aluminium phosphate has been shown by the inventors to improve the level of adsorption of Hib and HBsAg onto aluminium phosphate, and to help in the prevention of the desorption of all antigens in the DTPa-Hib combination vaccine (and hence improve the stability of the antigens). A method of washing the aluminium phosphate adjuvant is described in the

### Examples.

Due to the above disadvantageous effect of phosphate anions in solution shown by the inventors, any buffer used in the combination vaccine to stabilise pH should preferably be cationic in nature. Most preferably L-histidine monohydrate/monochloride should be used.

In a further aspect according to the invention, there is provided a combination vaccine obtained by the method of the invention described above.

In addition, there is provided a combination vaccine comprising a conjugated capsular polysaccharide of *Haemophilus influenzae* B (Hib) and DTPa, characterised in that Hib is adsorbed onto aluminium phosphate and any free aluminium hydroxide does not cause significant interference with Hib.

The extra-washed aluminium phosphate method can also be used independently to increase the adsorption of antigens onto aluminium phosphate, and to decrease their subsequent desorption in a combination vaccine comprising DTPa, where the method comprises the following steps:
(i) washing aluminium phosphate adjuvant so that the free phosphate ion concentration is reduced to 3 mM or less (preferably less than 1 mM);
(ii) sterilising the washed aluminium phosphate of (i);
(iii) selecting antigens to be adsorbed onto aluminium phosphate adjuvant;
(iv) adsorbing said antigens onto the washed aluminium phosphate;
(v) where required, combining the antigens of the combination vaccine.
Preferably the phosphate ions are removed either by repeated centrifugation and dilution steps (see Example 8), or by further diafiltration steps than those currently carried out by aluminium phosphate manufacturers using an ultrafiltration device. Preferably, the sterilisation of the extra-washed adjuvant in step (ii) is carried out by autoclaving.

In addition, a combination vaccine is provided by this invention which comprises DTPa and aluminium phosphate adjuvant, characterised in that said aluminium phosphate adjuvant has been washed so that the free phosphate ion concentration is reduced to 3 mM or less.

Also an immunogenic composition is provided comprising aluminium phosphate adjuvant and an antigen, characterised in that said aluminium phosphate adjuvant has been washed so that the free phosphate ion concentration is reduced to 3 mM or less.

The following examples illustrate, but do not the limit the scope of, the invention.

### Examples

### Example 1: Preparation of a vaccine formulation using the 'All Aluminium Phosphate' approach.

The method used to prepare DTPa-Hib-HBsAg-IPV liquid combination vaccine was as follows (quantities are scaled to represent one dose of vaccine):
1) 8 µg of PRN pre-adsorbed onto 0.05 mg of Superfos aluminium hydroxide was mixed with 40/8/32 DU of unadjuvanted IPV (type 1/2/3 respectively), and was stirred for over one hour at room temperature. The coating of IPV antigens stably maintained the aluminium hydroxide adjuvant in its antigen-saturated state in the final combination vaccine.
2) 17 Lf DT, 10 Lf TT, 25 µg PT, 25 µg FHA, and 10 µg HBsAg, all pre-adsorbed onto 0.17, 0.1, 0.05, 0.05, and 0.2 mg extra-washed Superfos aluminium phosphate respectively (expressed in amount of Al³⁺), were mixed along with the PRN from step 1) for over one hour at room temperature.
3) 10 µg Hib (PRP conjugated to tetanus toxoid) pre-adsorbed onto 0.12 mg extra-washed Superfos aluminium phosphate was added to the result of step 2), and the mixture was stirred for over 15 minutes at room temperature.
4) The mixture was made 10 mM for the cationic buffer L-histidine monohydrate/monochloride, and the pH was adjusted to 6.1 ± 0.1. Histidine has a pKa close to the desired final pH of 6.1 and, due to its cationic nature, does not provoke antigen desorption.
5) 5 mg/mL 2 phenoxyethanol were added to the mixture which was stirred for over 60 minutes at room temperature.
6) The pH of the mixture was checked as being 6.1 ± 0.1 (between 6 and 7 as specified by WHO for DT and DTP vaccines). The final mixed volume was 0.5 mL, and contained 140 mM NaCl.
7) The combination vaccine was stored for at least 2 weeks at room temperature prior to its use in immunogenic potency tests. The ratio of aluminium phosphate to aluminium hydroxide adjuvant (expressed as relative amounts of Al³⁺) present in the combination vaccine was 14:1.

DT, TT, PT, FHA, PRN, HbsAg, and Hib were adsorbed onto their respective adjuvants at pH 5.1 ± 0.1, 5.7 ± 0.1, 5.1 ± 0.1, 5.1 ± 0.1. 6.1 ± 0.1, 5.5 ± 0.1, and 5.1 ± 0.1 respectively.

### Example 2: Preparation of a vaccine formulation using the 'Light Aluminium Hydroxide' approach.

The method used to prepare DTPa-Hib-HBsAg-IPV liquid combination vaccine was as follows (quantities are scaled to represent one dose of vaccine):
1) 25 Lf DT, 10 Lf TT. 25 µg PT, 25 µg FHA, 8 µg PRN, and 40/8/32 DU IPV (type 1/2/3 respectively), all pre-adsorbed onto 0.1. 0.025, 0.005. 0.005, 0.05, and 0.01 mg Superfos aluminium hydroxide respectively (expressed in amount of Al³⁺), were mixed for over one hour at room temperature. With these quantities of aluminium hydroxide, the adjuvant was stably saturated with antigen.
2) 10 µg of HBsAg pre-adsorbed onto 0.2 mg of non-washed Superfos aluminium phosphate was mixed with the result of step 1) and was stirred for over one hour at room temperature.
3) 10 µg Hib (PRP conjugated to tetanus toxoid) pre-adsorbed onto 0.12 mg extra-washed Superfos aluminium phosphate was added to the result of step 2), and the mixture was stirred for over 15 minutes at room temperature.
4) The mixture was made 10 mM for the cationic buffer L-histidine monohydrate/monochloride, and the pH was adjusted to 6.1 ± 0.1. Histidine has a pKa close to the desired final pH of 6.1 and, due to its cationic nature, does not provoke antigen desorption.
5) 5 mg/mL 2 phenoxyethanol were added to the mixture which was stirred for over 60 minutes at room temperature.
6) The pH of the mixture was checked as being 6.1 ± 0.1 (between 6 and 7 as specified by WHO for DT and DTP vaccines). The final mixed volume was 0.5 mL, and contained 140 mM NaCl.
7) The combination vaccine was stored for at least 2 weeks at 4 °C prior to its use in immunogenic potency tests. The ratio of aluminium phosphate to aluminium hydroxide adjuvant (expressed as relative amounts of Al³⁺) present in the combination vaccine was 1.7:1.

DT, TT, PT, FHA, PRN, HbsAg, IPV, and Hib were adsorbed onto their respective adjuvants at pH 6.1 ± 0.1, 6.1 ± 0.1, 6.1 ± 0.1, 6.1 ± 0.1, 6.1 ± 0.1, 5.5 ± 0.1, 6.1 ± 0.1, and 5.1 ± 0.1 respectively.

### Example 3: Preparation of a vaccine formulation using an 'Hybrid All Aluminium Phosphate / Light Hydroxide' approach.

The method used to prepare DTPa-Hib-HBsAg-IPV combination vaccine was as follows (quantities are scaled to represent one dose of vaccine):
1) 25 Lf DT, 25 µg PT, 25 µg FHA, and 8 µg of PRN, all pre-adsorbed onto 0.1, 0.05, 0.05, and 0.05 mg of Superfos aluminium hydroxide respectively were mixed with 40/8/32 DU of unadjuvanted IPV (type 1/2/3 respectively).
2) 10 Lf TT. and 10 µg HBsAg, pre-adsorbed onto 0.25, and 0.2 mg non-washed Superfos aluminium phosphate respectively (expressed in amount of Al³⁺), were mixed together with the mixture from step 1) and stirred for over fifteen minutes at room temperature.
3) The mixture was made 10 mM for the cationic buffer L-histidine monohydrate/monochloride. This was stirred for over fifteen minutes at room temperature, and then the pH was adjusted to 6.1 ± 0.1. Histidine has a pKa close to the desired final pH of 6.1 and, due to its cationic nature, does not provoke antigen desorption.
4) 5 mg/mL 2 phenoxyethanol were added to the mixture which was stirred for over 60 minutes at room temperature.
5) The pH of the mixture was checked as being 6.1 ± 0.1 (between 6 and 7 as specified by WHO for DT and DTP vaccines). The final mixed volume was 0.5 mL, and contained 140 mM NaCl.
6) The combination vaccine was stored for at least 3 weeks at 4 °C prior to its use in immunogenic potency tests.
7) 10 µg Hib (PRP conjugated to tetanus toxoid) was extemporaneously added to the result of step 6).

DT, TT, PT, FHA, PRN, and HbsAg were adsorbed onto their respective adjuvants at pH 6.1 ± 0.1. 5.7 ± 0.1. 6.1 ± 0.1, 6.1 ± 0.1, 6.1 ± 0.1, and 5.3 ± 0.1 respectively.

### Example 4: Preparation of a vaccine formulation using an 'Adapted All Aluminium Phosphate / Light Hydroxide' approach.

The method used to prepare DTPa-Hib-HBsAg-IPV combination vaccine was as follows (quantities are scaled to represent one dose of vaccine):
1) 25 Lf DT was adsorbed onto 0.15 mg non-washed (normal) Superfos aluminium phosphate (expressed in amount of Al³⁻). This was added to 0.1 mg of Superfos aluminium hydroxide and the mixture was stirred for more than 30 minutes at room temperature. The pH was adjusted to 6.1 ± 0.1.
2) 25 µg PT, 25 µg FHA, and 8 µg of PRN, all pre-adsorbed onto 0.05, 0.05, and 0.05 mg of Superfos aluminium hydroxide respectively were mixed with the DT of step 1) and 40/8/32 DU of unadjuvanted IPV (type 1/2/3 respectively).
3) 10 Lf TT, and 10 µg HBsAg, pre-adsorbed onto 0.1. and 0.2 mg non-washed (normal) Superfos aluminium phosphate respectively (expressed in amount of Al³⁺), were mixed together with the mixture from step 2) and stirred for over fifteen minutes at room temperature.
4) The mixture was made 10 mM for the cationic buffer L-histidine monohydrate/monochloride, and the pH was adjusted to 6.1 ± 0.1. Histidine has a pKa close to the desired final pH of 6.1 and, due to its cationic nature, does not provoke antigen desorption.
5) 5 mg/mL 2 phenoxyethanol were added to the mixture which was stirred for over 60 minutes at room temperature.
6) The pH of the mixture was checked as being 6.1 ± 0.1 (between 6 and 7 as specified by WHO for DT and DTP vaccines). The final mixed volume was 0.5 mL, and contained 140 mM NaCl.
7) The combination vaccine was stored for at least 3 weeks at 4 °C prior to its use in immunogenic potency tests.
8) 10 µg Hib (PRP conjugated to tetanus toxoid) was extemporaneously added to the result of step 7).

DT, TT, PT, FHA, PRN, and HbsAg were adsorbed onto their respective adjuvants at pH 5.4 ± 0.1, 5.7 ± 0.1, 6.1 ± 0.1, 6.1 ± 0.1, 6.1 ± 0.1, and 5.3 ± 0.1 respectively.

### Example 5: Interference measurements in clinical tests of the combination vaccines

The clinical tests below show the interference problem that was encountered on anti-PRP antibody titres in combination vaccines comprising DTPa (conventionally adsorbed onto an excess of aluminium hydroxide) and Hib.

Initially seronegative healthy baby volunteers were immunised with 3 doses of combination vaccine administered in the schedules given below. Responses were defined as subjects with antibody titres significantly above background. Titres were expressed in mIU/mL.

The results below are expressed as Geometric Mean Titres (GMT) in mIU/mL. For anti-PRP, the number in brackets represents the % of individuals that had GMTs over 1.0. The results show that the Hib interference problem could be alleviated using the 'All Aluminium Phosphate' approach without affecting the efficacy of the other antigens in the combination vaccine.

**TABLE I:**

| Combination | PT | FHA | PRN | DT | TT | HBsAg | PRP of Hib |
|---|---|---|---|---|---|---|---|
| DTPa + Hib* | 47.7(100) | 182.5(100) | 130.8(100) | 1.32(100) | 1.21(100) | NA | 6.09(94.4) |
| DTPaHepB/Hib** | 27.0(100) | 137.7(100) | 101.7(100) | 0.98(100) | 1.45(100) | 141.4(94.4) | 0.95(50.0) |
| DTPaHepB/Hib*** | 78.3(100) | 247.5(100) | 112.2(100) | 1.73(100) | 1.74(100) | 374.6(90.0) | 5.01(95.0) |
| DTPaHepB/Hib† | 47.2(100) | 210.8(100) | 152.6(100) | 1.82(100) | 1.64(100) | 454.5(96.2) | 3.1(80.8) |
| DTPaHepB/Hib†† | 96(100) | 176.1(100) | 281.5(100) | 2.97(100) | 3.1(100) | 590.5(92.3) | 9.67(96.2) |

| | | | | | | | |
|---|---|---|---|---|---|---|---|
| * - Reference for PRP(Hib) antibody titre - DTPa in one arm and Hiberix^{™} (non-adjuvanted PRP conjugated to tetanus toxoid) (SmithKline Beecham Biologicals) in the other arm. DTPa was adsorbed onto 500 µg/mL aluminium hydroxide. | | | | | | | |
| ** - DTPaHepB in combination with Hiberix^{™}. Lyophilised Hib was extemporaneously reconstituted with DTPa-HepB vaccine. DTPa was adsorbed onto 1000 µg/mL aluminium hydroxide. HepB - the HBsAg was adsorbed onto aluminium phosphate and then combined with DTPa as described in WO 93/24148. | | | | | | | |
| *** - The 'All Aluminium Phosphate' approach with unadjuvanted Hib. DTPa-HepB was adsorbed onto aluminium phosphate (except for pertactin that was adsorbed onto aluminium hydroxide) using the method of Example 1 except that Hib was not added. Lyophilised, unadjuvanted Hib (Hiberix^{™}) was extemporaneously reconstituted with the resulting DTPa-HepB vaccine. The above group of 3 experiments was done on a group of 18. Individuals were vaccinated at the age of 3, 4, and 5 months, with 0.5 mL doses. | | | | | | | |
| † - DTPaHepB in combination with Hiberix^{™}. Lyophilised Hib was extemporaneously reconstituted with DTPa-HepB vaccine. DTPa was adsorbed onto 500 µg/mL aluminium hydroxide. HepB - the HBsAg was adsorbed onto aluminium phosphate and then combined with DTPa as described in WO 93/24148. | | | | | | | |
| †† - The 'All Aluminium Phosphate' approach with unadjuvanted Hib. DTPa-HepB was adsorbed onto aluminium phosphate (except for pertactin that was adsorbed onto aluminium hydroxide) using the method of Example 1 except that Hib was not added. Lyophilised, unadjuvanted Hib (Hiberix^{™}) was extemporaneously reconstituted with the resulting DTPa-HepB vaccine. The above group of 2 experiments was done in Turkey on a group of 30 individuals. Individuals were vaccinated at the age of 3, 4, and 5 months, with 0.5 mL doses. | | | | | | | |

### Example 6: Mouse Pertussis Lung Clearance Activity of the 'Light Aluminium Hydroxide' and 'All Aluminium Phosphate' Formulations

'Light Aluminium Hydroxide' and 'All Aluminium Phosphate' Formulations without Hib were tested shortly after being formulated to examine their pertussis mouse lung clearance activity as compared with a commercial DTPa vaccine formulation (INFANRIX^{™} - SmithKline Beecham Biologicals). The addition of unadjuvanted Hib to the formulations was assessed for its effect on the activity.

The assays were done with a two dose immunization schedule (using a one quarter human dose). Immunised mice were infected with *Bordetella pertussis,* and the CFU/lungs of bacteria was measured against time post challenge.

The results can be seen in Figure 1. The pertussis animal challenge data show that pertussis lung clearance activity is equivalent using the 'Light Aluminium Hydroxide' and 'All Aluminium Phosphate' Formulations (with or without unadjuvanted Hib) as compared with previous classic DTPa formulations.

### Example 7: Infant Rat Hib Immunogenicity Studies with 'Light Aluminium Hydroxide' and 'All Aluminium Phosphate' Formulations

An infant rat model was used to assess the immunogenicity of the Hib component in 'Light Aluminium Hydroxide' and 'All Aluminium Phosphate' vaccine formulations. The experiments were done with 1/10 human dose (50 µL injection) being administered subcutaneously with a 0-14-28 day schedule (starting day 7 after birth) with blood samples taken at day 42. 24-30 animals were assessed per group. Hib adsorbed onto aluminium phosphate was either added to the formulation (the 'liquid' formulation of Examples 1 and 2) two weeks before the experiments, or added extemporaneously. Geometric Mean antibody Titres (GMT) against PRP were measured.

Results indicated that PRP titres for both the 'Light Aluminium Hydroxide' and 'All Aluminium Phosphate' formulations (whether in a 'liquid' formulation or in an extemporaneously-prepared formulation) were equivalent or at most minimally interfered compared with the titres measured after the administration of monovalent Hib adsorbed onto aluminium phosphate.

### Example 8: Preparation of 'Extra-Washed Aluminium Phosphate Adjuvant'

To wash the aluminium phosphate, the following steps were carried out: the aluminium phosphate solution was centrifuged at 3500 rpm for 10 minutes, and the supernatant was collected. The pellet was resuspended with 150 mM NaCl. These steps were repeated at least 3-4 times. The result of this was filtered through a 100 µm filter (to remove potential aggregates), and the aluminium phosphate was sterilised by autoclaving. The sterile adjuvant was stored at 4 °C until it was used. Extra-washed and autoclaved aluminium phosphate is stable for at least 45 days at room temperature.

Superfos aluminium phosphate was used for this process. The product provided by the manufacturer contains up to 5 mM free phosphate ion. When thoroughly washed the free phosphate concentration can reduce to less than 0.5 mM, which rises to 1 mM if the washed solution is incubated at 37 °C for 7 days. If the washed aluminium phosphate is subsequently autoclaved, the free phosphate concentration can rise to 2.0-3.0 mM.

### Results:

The effect of washing aluminium phosphate on the adsorption of antigens (H = hours; RT = room temperature) is shown in Table II.

**TABLE II**

| Antigen (adsorbed) | Dose (Unit/mg Al) | Adsorption Time | Adjuvant Status | [PO₄] in Supernatent | % Adsorption |
|---|---|---|---|---|---|
| DT | 25 Lf/0.25 | 48H RT pH5.1 | Not washed | 4.5 mM | 70 |
| DT | 25 Lf/0.25 | 24H RT pH5.1 | Washed | 0.5 mM | 93 |
| DT | 25 Lf/0.25 | 24H RT pH5.1 | Washed and Autoclaved | 1.8 mM | 90 |
| TT | 10 Lf/0.10 | 48H RT pH5.7 | Not washed | 4.5 mM | 84 |
| TT | 10 Lf/0.10 | 24H RT pH5.7 | Washed and Autoclaved | 1.8 mM | 99 |
| Hib[on TT] | 10 µg/0.12 | 16H RT pH5.1 | Not washed | 4.5 mM | 92 |
| Hib[on TT] | 10 µg/0.12 | 16H RT pH5.1 | Washed and Autoclaved | 1.8 mM | 100 |
| HBsAg | 10 µg/0.02 | 48H RT pH5.5 | Washed | 0.33 mM | 97.4 |
| HBsAg | 10 µg/0.02 | 48H RT pH5.5 | Washed and Autoclaved | 0.85 mM | 96.7 |
| HBsAg | 10 µg/0.02 | 48H RT pH5.5 | Not washed | 1.73 mM | 95.6 |
| HBsAg | 10 µg/0.02 | 48H RT pH5.5 | Added PO₄ | 6.66 mM | 85.3 |

As can be seen from the results, the use of extra-washed aluminium phosphate improves the adsorption of all the antigens, especially for DT and TT. It can also be seen that to add free phosphate anions to the adsorption procedure results in a significant decrease in the adsorption of HBsAg.

### Example 9: The effect of unadsorbed antigens and free phosphate on the desorption of PRN:

PRN should be adsorbed onto aluminium hydroxide in order to stably adsorb antigen at pH 6.1 (and retain potency) as described above. When dealing with small amounts of adjuvant (in order for it to be pre-saturated with antigen) it is important to examine the stability of the antigen in a combination vaccine to determine if the aluminium hydroxide is over-saturated in the context of the combination. The following table shows the effect of mixing PRN pre-adsorbed on aluminium hydroxide (8 µg per 10µg or 50µg Al³⁺) with one dose of a non pre-adsorbed antigen (or aluminium phosphate) over 7 days at 4 °C or 37 °C, pH 6.1. Final antigen concentrations were equivalent to their concentrations in a typical combination vaccine. The % PRN in the supernatant was measured using ELISA; the PRN was measured in the supernatant and in the pellet (500 mM phosphate anions were used to desorb the PRN and the amount of released PRN was measured). A % was then calculated.

**TABLE III:**

| Second Antigen (non-adsorbed) | %PRN in supernatant - 10µg Al - 4 °C | %PRN in supernatant - 10µg Al - 37 °C | %PRN in supernatant - 50µg Al - 4 °C | %PRN in supernatant -50µg Al - 37 °C |
|---|---|---|---|---|
| PRN | <0.1 | / | / | / |
| PT | <0.1 | <0.1 | / | / |
| FHA | <0.1 | <0.1 | / | / |
| HBsAg | 3.8 | 0.2 | / | / |
| Hib | 4.4 | 62 | 0.4 | 0.3 |
| IPV | 7.5 | 56 | <0.1 | 1.6 |
| TT | 5.2 | 62 | / | / |
| DT | 28 | 80 | / | / |
| 1 mg/mL non-washed AlPO₄ | / | 44 | / | / |

As the table shows, in the combination vaccine context, the aluminium hydroxide is over saturated with 8 µg PRN using 10 µg of aluminium hydroxide, but is stable using 50 µg of the adjuvant.

Also the effect of free aluminium phosphate is shown to cause some desorption of the PRN. The use of extra-washed Aluminium phosphate would limit this effect.

## Claims

1. A method to reduce interference of a capsular polysaccharide component of a conjugated *Haemophilus influenzae* B vaccine (Hib) in a combination vaccine comprising DTPa, wherein such method comprises:
(i) selecting one or more antigen(s) to be adsorbed onto aluminium hydroxide adjuvant;
(ii) pre-saturating the aluminium hydroxide adjuvant with the selected antigens such that there is a minimisation of any interference to Hib antigen in the combination vaccine caused by aluminium hydroxide adjuvant;
(iii) selecting Hib plus one or more additional antigen(s) to be adsorbed onto aluminium phosphate;
(iv) adsorbing Hib and said additional antigens onto aluminium phosphate;
(v) combining all antigens in said vaccine.

2. The method of claim 1 wherein the Hib adsorbed onto aluminium phosphate is mixed extemporaneously with the other antigens of the combination vaccine.

3. The method of claims 1 to 2 wherein the combination vaccine additionally comprises one or more antigens selected from the group: Hepatitis B surface antigen (HBsAg), inactivated Hepatitis A virus, inactivated Polio virus, *N. meningitidis* A capsular polysaccharide, *N. meningitidis* C capsular polysaccharide, *Streptococcus pneumoniae* capsular polysaccharide, *Streptococcus pneumoniae* proteins, *Moraxella catarrhalis* outer membrane proteins, non-typeable *Haemophilus influenzae* outer membrane proteins, *N. meningitidis* B outer membrane proteins.

4. The method of claim 3 wherein the combination vaccine comprises Hepatitis B surface antigen adsorbed onto aluminium phosphate.

5. The method of any one of claims 1 to 4 wherein the ratio of aluminium phosphate to aluminium hydroxide adjuvant present in the combination vaccine ranges from 1:1 to 20:1.

6. The method of any one of claims 1 to 5 wherein all the antigens of the combination vaccine are adsorbed onto aluminium phosphate, with the proviso that pertactin is adsorbed onto aluminium hydroxide.

7. The method of claim 6 wherein the pertactin adsorbed onto aluminium hydroxide is combined with unadsorbed inactivated Polio virus antigens before being combined with the aluminium phosphate adsorbed antigens.

8. The method of any one of claims 1 to 5 wherein all the antigens of the combination vaccine are adsorbed onto aluminium hydroxide, with the proviso that HBsAg and Hib are adsorbed onto aluminium phosphate.

9. The method of claim 8 wherein the antigens adsorbed onto aluminium hydroxide are combined before the HBsAg antigen, adsorbed onto aluminium phosphate, is added and Hib, adsorbed onto aluminium phosphate, is combined after the HBsAg antigen has been added.

10. The method of claims 8 to 9 wherein additional free aluminium phosphate is added to the combination vaccine.

11. A method to reduce interference of a capsular polysaccharide component of a conjugated *Haemophilus influenzae* B vaccine (Hib) in a combination vaccine comprising DTPa, wherein such method comprises:
(i) selecting one or more antigen(s) to be adsorbed onto aluminium hydroxide adjuvant;
(ii) pre-saturating the aluminium hydroxide adjuvant with the selected antigens such that there is a minimisation of any interference to Hib antigen in the combination vaccine caused by aluminium hydroxide adjuvant;
(iii) selecting one or more additional antigen(s) to be adsorbed onto aluminium phosphate;
(iv) adsorbing said additional antigens onto aluminium phosphate;
(vi) combining all antigens in said vaccine with unadjuvanted Hib.

12. A method to reduce interference of a capsular polysaccharide component of a conjugated *Haemophilus influenzae* B vaccine (Hib) in a combination vaccine comprising DTPa, wherein such method comprises:
(i) selecting one or more antigen(s) to be adsorbed onto aluminium hydroxide adjuvant;
(ii) pre-saturating the aluminium hydroxide adjuvant with the selected antigens such that there is a minimisation of any interference to Hib antigen in the combination vaccine caused by aluminium hydroxide adjuvant;
(iii) selecting one or more additional antigen(s) to be adsorbed onto aluminium phosphate;
(iv) adsorbing said additional antigens onto aluminium phosphate;
(v) combining all antigens in said vaccine;
(vi) extemporaneously adding either unadjuvanted Hib or Hib adsorbed onto aluminium phosphate.

13. The method of claim 12 wherein the combination vaccine additionally comprises one or more antigens selected from the group: Hepatitis B surface antigen (HBsAq), inactivated Hepatitis A virus, inactivated Polio virus, *N. meningitidis* A capsular polysaccharide, *N. meningitidis* C capsular polysaccharide, *Streptococcus pneumoniae* capsular polysaccharide, *Streptococcus pneumoniae* proteins, *Moraxella catarrhalis* outer membrane proteins, non-typeable *Haemophilus influenzae* outer membrane proteins, *N. meningitidis* B outer membrane proteins.

14. The method of claim 13 wherein the combination vaccine comprises Hepatitis B surface antigen adsorbed onto aluminium phosphate.

15. The method of any one of claims 12 to 14 wherein the ratio of aluminium phosphate to aluminium hydroxide adjuvant present in the combination vaccine ranges from 1:1 to 20:1.

16. The method of any one of claims 12 to 15 wherein all the antigens of the combination vaccine are adsorbed onto aluminium phosphate, with the proviso that pertactin is adsorbed onto aluminium hydroxide and that Hib is added either unadjuvanted or adsorbed onto aluminium phosphate.

17. The method of claim 16 wherein the pertactin adsorbed onto aluminium hydroxide is combined with unadsorbed inactivated Polio virus antigens before being combined with the aluminium phosphate adsorbed antigens.

18. The method of any one of claims 12 to 15 wherein all the antigens of the combination vaccine are adsorbed onto aluminium phosphate, with the proviso that pertactin, diphtheria toxoid, pertussis toxoid, filamentous haemagglutinin are adsorbed onto aluminium hydroxide and that Hib is added either unadjuvanted or adsorbed onto aluminium phosphate.

19. The method of claim 18 wherein the pertactin, diphtheria toxoid, pertussis toxoid, filamentous haemagglutinin adsorbed onto aluminium hydroxide are combined with unadsorbed inactivated Polio virus antigens before being combined with the aluminium phosphate adsorbed antigens.

20. The method of any one of claims 12 to 15 wherein all the antigens of the combination vaccine are adsorbed onto aluminium hydroxide, with the proviso that HBsAg is adsorbed onto aluminium phosphate and that Hib is added either unadjuvanted or adsorbed onto aluminium phosphate.

21. The method of claim 20 wherein the antigens adsorbed onto aluminium hydroxide are combined before the HBsAg antigen, adsorbed onto aluminium phosphate, is added.

22. The method of claims 20 to 21 wherein additional free aluminium phosphate is added to the combination vaccine before the extemporaneous addition of unadjuvanted Hib.

23. The method of any one of claims 12 to 22 wherein one or more of the antigens selected to pre-saturate the aluminium hydroxide has been previously adsorbed onto aluminium phosphate.

24. The method of claim 23 wherein diphtheria toxoid adsorbed onto aluminium phosphate is one of the antigens selected to pre-saturate the aluminium hydroxide.

25. The method of any one of claims 1 to 24 wherein the combination vaccine is buffered with L-histidine.

26. A combination vaccine comprising:
a) DTPa and
b) Hib adsorbed to aluminium phosphate,
obtainable by the method of any one of claims 1 to 25.

## Patentansprüche

1. Verfahren zur Reduzierung der Interferenz einer Kapselpolysaccharidkomponente eines konjugierten Haemophilus influenzae B-Impfstoffs (Hib) in einem Kombinationsimpfstoff, der DTPa umfaßt, worin ein solches Verfahren die folgenden Schritte umfaßt:
(i) Auswählen eines oder mehrerer Antigene zur Adsorption an Aluminiumhydroxid-Hilfsstoff;
(ii) Vorsättigen des Aluminiumhydroxid-Hilfsstoffs mit den ausgewählten Antigenen, so daß es eine Minimierung jeglicher Interferenz gegenüber Hib-Antigen im Kombinationsimpfstoff gibt, die durch Aluminiumhydroxid-Hilfsstoff verursacht wird;
(iii) Auswählen von Hib plus einem oder mehreren zusätzlichen Antigenen zur Adsorption an Aluminiumphosphat;
(iv) Adsorbieren von Hib und den zusätzlichen Antigenen an Aluminiumphosphat;
(v) Kombinieren aller Antigene im Impfstoff.

2. Verfahren gemäß Anspruch 1, worin das an Aluminiumphosphat adsorbierte Hib unvorbereitet mit den anderen Antigenen des Kombinationsimpfstoffs vermischt wird.

3. Verfahren gemäß Ansprüchen 1 bis 2, worin der Kombinationsimpfstoff zusätzlich ein oder mehrere Antigene umfaßt, die aus der folgenden Gruppe ausgewählt sind: Hepatitis B-Oberflächenantigen (HBsAg), inaktiviertes Hepatitis A-Virus, inaktiviertes Poliovirus, N. meningitidis A-Kapselpolysaccharid, N. meningitidis C-Kapselpolysaccharid, Streptococcus pneumoniae-Kapselpolysaccharid, Streptococcus pneumoniae-Proteine, Moraxella catarrhalis-Außenmembranproteine, nicht-typisierbare Haemophilus influenzae-Außenmembranproteine, N. meningitidis B-Außenmembranproteine.

4. Verfahren gemäß Anspruch 3, worin der Kombinationsimpfstoff an Aluminiumphosphat adsorbiertes Hepatitis B-Oberflächenantigen umfaßt.

5. Verfahren gemäß einem der Ansprüche 1 bis 4, worin das Verhältnis von Aluminiumphosphat zu im Kombinationsimpfstoff vorhandenem Aluminiumhydroxid-Hilfsstoff von 1:1 bis 20:1 reicht.

6. Verfahren gemäß einem der Ansprüche 1 bis 5, worin alle Antigene des Kombinationsimpfstoffs an Aluminiumphosphat adsorbiert werden, mit der Maßgabe, daß Pertactin an Aluminiumhydroxid adsorbiert wird.

7. Verfahren gemäß Anspruch 6, worin das an Aluminiumhydroxid adsorbierte Pertactin mit nicht-adsorbierten inaktivierten Poliovirus-Antigenen vor der Kombination mit den an Aluminiumphosphat adsorbierten Antigenen kombiniert wird.

8. Verfahren gemäß einem der Ansprüche 1 bis 5, worin alle Antigene des Kombinationsimpfstoffs an Aluminiumhydroxid adsorbiert werden, mit der Maßgabe, daß HBsAg und Hib an Aluminiumphosphat adsorbiert werden.

9. Verfahren gemäß Anspruch 8, worin die an Aluminiumhydroxid adsorbierten Antigene kombiniert werden, bevor das an Aluminiumphosphat adsorbierte HBsAg-Antigen hinzugegeben wird, und an Aluminiumphosphat adsorbiertes Hib kombiniert wird, nachdem das HBsAg-Antigen hinzugegeben wurde.

10. Verfahren gemäß Ansprüchen 8 bis 9, worin zusätzliches freies Aluminiumphosphat zum Kombinationsimpfstoff hinzugegeben wird.

11. Verfahren zur Reduzierung der Interferenz einer Kapselpolysaccharidkomponente eines konjugierten Haemophilus influenzae B-Impfstoffs (Hib) in einem Kombinationsimpfstoff, der DTPa umfaßt, worin ein solches Verfahren die folgenden Schritte umfaßt:
(i) Auswählen eines oder mehrerer Antigene zur Adsorption an Aluminiumhydroxid-Hilfsstoff;
(ii) Vorsättigen des Aluminiumhydroxid-Hilfsstoffs mit den ausgewählten Antigenen, so daß es eine Minimierung jeglicher Interferenz gegenüber Hib-Antigen im Kombinationsimpfstoff gibt, die durch Aluminiumhydroxid-Hilfsstoff verursacht wird;
(iii) Auswählen eines oder mehrerer zusätzlicher Antigene zur Adsorption an Aluminiumphosphat;
(iv) Adsorbieren der zusätzlichen Antigene an Aluminiumphosphat;
(v) Kombinieren aller Antigene im Impfstoff mit Hib ohne Hilfsstoff.

12. Verfahren zur Reduzierung der Interferenz einer Kapselpolysaccharidkomponente eines konjugierten Haemophilus influenzae B-Impfstoffs (Hib) in einem Kombinationsimpfstoff, der DTPa umfaßt, worin ein solches Verfahren die folgenden Schritte umfaßt:
(i) Auswählen eines oder mehrerer Antigene zur Adsorption an Aluminiumhydroxid-Hilfsstoff;
(ii) Vorsättigen des Aluminiumhydroxid-Hilfsstoffs mit den ausgewählten Antigenen, so daß es eine Minimierung jeglicher Interferenz gegenüber Hib-Antigen im Kombinationsimpfstoff gibt, die durch Aluminiumhydroxid-Hilfsstoff verursacht wird;
(iii) Auswählen eines oder mehrerer zusätzlicher Antigene zur Adsorption an Aluminiumphosphat;
(iv) Adsorbieren der zusätzlichen Antigene an Aluminiumphosphat;
(v) Kombinieren aller Antigene im Impfstoff;
(vi) unvorbereitetes Zugeben von entweder Hib ohne Hilfsstoff oder von an Aluminiumphosphat adsorbiertem Hib.

13. Verfahren gemäß Anspruch 12, worin der Kombinationsimpfstoff zusätzlich ein oder mehrere Antigene umfaßt, die aus der folgenden Gruppe ausgewählt sind: Hepatitis B-Oberflächenantigen (HBsAg), inaktiviertes Hepatitis A-Virus, inaktiviertes Poliovirus, N. meningitidis A-Kapselpolysaccharid, N. meningitidis C-Kapselpolysaccharid, Streptococcus pneumoniae-Kapselpolysaccharid, Streptococcus pneumoniae-Proteine, Moraxella catarrhalis-Außenmembranproteine, nicht-typisierbare Haemophilus influenzae-Außenmembranproteine, N. meningitidis B-Außenmembranproteine.

14. Verfahren gemäß Anspruch 13, worin der Kombinationsimpfstoff an Aluminiumphosphat adsorbiertes Hepatitis B-Oberflächenantigen umfaßt.

15. Verfahren gemäß einem der Ansprüche 12 bis 14, worin das Verhältnis von Aluminiumphosphat zu im Kombinationsimpfstoff vorhandenem Aluminiumhydroxid-Hilfsstoff von 1:1 bis 20:1 reicht.

16. Verfahren gemäß einem der Ansprüche 12 bis 15, worin alle Antigene des Kombinationsimpfstoffs an Aluminiumphosphat adsorbiert werden, mit der Maßgabe, daß Pertactin an Aluminiumhydroxid adsorbiert wird und daß Hib entweder ohne Hilfsstoff oder adsorbiert an Aluminiumphosphat hinzugegeben wird.

17. Verfahren gemäß Anspruch 16, worin das an Aluminiumhydroxid adsorbierte Pertactin mit nicht-adsorbierten inaktivierten Poliovirus-Antigenen vor der Kombination mit den an Aluminiumphosphat adsorbierten Antigenen kombiniert wird.

18. Verfahren gemäß einem der Ansprüche 12 bis 15, worin alle Antigene des Kombinationsimpfstoffs an Aluminiumphosphat adsorbiert werden, mit der Maßgabe, daß Pertactin, Diphtherietoxoid, Pertussistoxoid, filamentöses Hämagglutinin an Aluminiumhydroxid adsorbiert werden und daß Hib entweder ohne Hilfsstoff oder adsorbiert an Aluminiumphosphat hinzugegeben wird.

19. Verfahren gemäß Anspruch 18, worin das an Aluminiumhydroxid absorbierte Pertactin, Diphtherietoxoid, Pertussistoxoid, filamentöse Hämagglutinin mit nicht-adsorbierten inaktivierten Poliovirus-Antigenen vor der Kombination mit den an Aluminiumphosphat adsorbierten Antigenen kombiniert werden.

20. Verfahren gemäß einem der Ansprüche 12 bis 15, worin alle Antigene des Kombinationsimpfstoffs an Aluminiumhydroxid adsorbiert werden, mit der Maßgabe, daß HBsAg an Aluminiumphosphat adsorbiert wird und daß Hib entweder ohne Hilfsstoff oder adsorbiert an Aluminiumphosphat hinzugegeben wird.

21. Verfahren gemäß Anspruch 20, worin die an Aluminiumhydroxid adsorbierten Antigene kombiniert werden, bevor das an Aluminiumphosphat adsorbierte HBsAg-Antigen hinzugegeben wird.

22. Verfahren gemäß Ansprüchen 20 bis 21, worin zusätzliches freies Aluminiumphosphat zum Kombinationsimpfstoff vor der unvorbereiteten Zugabe von Hib ohne Hilfsstoff hinzugegeben wird.

23. Verfahren gemäß einem der Ansprüche 12 bis 22, worin eines oder mehrere der Antigene, die zur Vorsättigung des Aluminiumhydroxids ausgewählt werden, zuvor an Aluminiumphosphat adsorbiert wurden.

24. Verfahren gemäß Anspruch 23, worin an Aluminiumphosphat adsorbiertes Diphtherietoxoid eines der Antigene ist, die zur Vorsättigung des Aluminiumhydroxids ausgewählt werden.

25. Verfahren gemäß einem der Ansprüche 1 bis 24, worin der Kombinationsimpfstoff mit L-Histidin gepuffert wird.

26. Kombinationsimpfstoff, der
a) DTPa und
b) an Aluminiumphosphat adsorbiertes Hib umfaßt,
erhältlich durch das Verfahren gemäß einem der Ansprüche 1 bis 25.

## Revendications

1. Procédé pour réduire l'interférence d'un composant polysaccharide capsulaire d'un vaccin *Heamophilus influenza* B (Hib) conjugué dans un vaccin combiné, comprenant DTPa, où le procédé comprend :
(i) la sélection d'un ou de plusieurs antigènes à adsorber sur un adjuvant hydroxyde d'aluminium ;
(ii) la présaturation de l'adjuvant hydroxyde d'aluminium avec les antigènes sélectionnés de sorte qu'il y a minimisation de toute interférence avec l'antigène Hib dans le vaccin combiné, provoquée par l'adjuvant hydroxyde d'aluminium ;
(iii) la sélection de Hib plus un ou plusieurs antigènes à adsorber sur le phosphate d'aluminium ;
(iv) l'adsorption du Hib et desdits antigènes supplémentaires sur le phosphate d'aluminium ;
(v) la combinaison de tous les antigènes dans ledit vaccin.

2. Procédé selon la revendication 1, dans lequel le Hib adsorbé sur le phosphate d'aluminium est mélangé de manière extemporanée, avec les autres antigènes du vaccin combiné.

3. Procédé selon les revendications 1 et 2, dans lequel le vaccin combiné comprend en outre, un ou plusieurs antigènes sélectionnés parmi le groupe : antigène de surface de l'hépatite B (HBsAg), virus de l'hépatite A inactivé, virus de la polio inactivé, polysaccharide capsulaire de *N*. *meningitidis* A, polysaccharide capsulaire de N. meningitidis C, polysaccharide capsulaire de *Streptococcus pneumoniae,* protéines de *Streptococcus pneumoniae*, protéines de membrane externe de *Moraxella catarrhalis*, protéines de membrane externe de *Haemophilus influenza* non typable, protéines de membrane externe de *N*. *meningitidis* B.

4. Procédé selon la revendication 3, dans lequel le vaccin combiné comprend l'antigène de surface de l'hépatite B, adsorbé sur le phosphate d'aluminium.

5. Procédé selon l'une quelconque des revendications 1 à 4, dans lequel le rapport du phosphate d'aluminium à l'adjuvant hydroxyde d'aluminium présents dans le vaccin combiné se situe dans l'intervalle allant de 1:1 à 20:1.

6. Procédé selon l'une quelconque des revendications 1 à 5, dans lequel tous les antigènes du vaccin combiné sont adsorbés sur le phosphate d'aluminium, avec la condition que la pertactine est adsorbée sur l'hydroxyde d'aluminium.

7. Procédé selon la revendication 6, dans lequel la pertactine adsorbée sur l'hydroxyde d'aluminium est combinée aux antigènes du virus de la polio, non adsorbés, avant d'être combinée aux antigènes adsorbés sur le phosphate d'aluminium.

8. Procédé selon l'une quelconque des revendications 1 à 5, dans lequel tous les antigènes du vaccin combiné sont adsorbés sur l'hydroxyde d'aluminium, avec la condition que HBsAg et Hib sont adsorbés sur le phosphate d'aluminium.

9. Procédé selon la revendication 8, dans lequel les antigènes adsorbés sur l'hydroxyde d'aluminium sont combinés avant que l'antigène HBsAg, adsorbé sur le phosphate d'aluminium, soit ajouté et Hib, adsorbé sur le phosphate d'aluminium, est combiné après que l'antigène HBsAg a été ajouté.

10. Procédé des revendications 8 et 9, où du phosphate d'aluminium libre supplémentaire est ajouté au vaccin combiné.

11. Procédé pour réduire l'interférence d'un composant polysaccharide capsulaire d'un vaccin *Heamophilus influenza* B (Hib) conjugué dans un vaccin combiné, comprenant DTPa, où le procédé comprend :
(i) la sélection d'un ou de plusieurs antigènes à adsorber sur un adjuvant hydroxyde d'aluminium ;
(ii) la présaturation de l'adjuvant hydroxyde d'aluminium avec les antigènes sélectionnés de sorte qu'il y a minimisation de toute interférence avec l'antigène Hib dans le vaccin combiné, provoquée par l'adjuvant hydroxyde d'aluminium ;
(iii) la sélection d'un ou de plusieurs antigènes supplémentaires à adsorber sur le phosphate d'aluminium ;
(iv) l'adsorption desdits antigènes supplémentaires sur le phosphate d'aluminium ;
(v) la combinaison de tous les antigènes dans ledit vaccin avec le Hib non adjuvé.

12. Procédé pour réduire l'interférence d'un composant polysaccharide capsulaire d'un vaccin *Heamophilus influenza* B (Hib) conjugué dans un vaccin combiné, comprenant DTPa, où le procédé comprend :
(i) la sélection d'un ou de plusieurs antigènes à adsorber sur un adjuvant hydroxyde d'aluminium ;
(ii) la présaturation de l'adjuvant hydroxyde d'aluminium avec les antigènes sélectionnés de sorte qu'il y a minimisation de toute interférence avec l'antigène Hib dans le vaccin combiné, provoquée par l'adjuvant hydroxyde d'aluminium ;
(iii) la sélection d'un ou de plusieurs antigènes supplémentaires à adsorber sur le phosphate d'aluminium ;
(iv) l'adsorption desdits antigènes supplémentaires sur le phosphate d'aluminium ;
(v) la combinaison de tous les antigènes dans ledit vaccin
(vi) l'addition extemporanée de Hib non adjuvé ou de Hib adsorbé sur phosphate d'aluminium.

13. Procédé selon la revendication 12, dans lequel le vaccin combiné comprend en outre, un ou plusieurs antigènes sélectionnés parmi le groupe : antigène de surface de l'hépatite B (HBsAg), virus de l'hépatite A inactivé, virus de la polio inactivé, polysaccharide capsulaire de *N*. *meningitidis* A, polysaccharide capsulaire de N. meningitidis C, polysaccharide capsulaire de *Streptococcus pneumoniae,* protéines de *Streptococcus pneumoniae,* protéines de membrane externe de *Moraxella catarrhalis,* protéines de membrane externe de *Haemophilus influenza* non typable, protéines de membrane externe de *N*. *meningitidis* B.

14. Procédé selon la revendication 13, dans lequel le vaccin combiné comprend l'antigène de surface de l'hépatite B, adsorbé sur le phosphate d'aluminium.

15. Procédé selon l'une quelconque des revendications 12 à 14, dans lequel le rapport du phosphate d'aluminium à l'adjuvant hydroxyde d'aluminium présents dans le vaccin combiné se situe dans l'intervalle allant de 1:1 à 20:1.

16. Procédé selon l'une quelconque des revendications 12 à 15, dans lequel tous les antigènes du vaccin combiné sont adsorbés sur le phosphate d'aluminium, avec la condition que la pertactine est adsorbée sur l'hydroxyde d'aluminium et que le Hib est ajouté sous forme non adjuvée ou adsorbé sur le phosphate d'aluminium.

17. Procédé selon la revendication 16, dans lequel la pertactine adsorbée sur l'hydroxyde d'aluminium est combinée aux antigènes du virus de la polio inactivé, non adsorbés, avant d'être combinée aux antigènes adsorbés sur le phosphate d'aluminium.

18. Procédé selon l'une quelconque des revendications 12 à 15, dans lequel tous les antigènes du vaccin combiné sont adsorbés sur le phosphate d'aluminium, avec la condition que la pertactine, le toxoïde de diphtérie, le toxoïde de pertussis, l'hémagglutinine filamenteuse sont adsorbés sur l'hydroxyde d'aluminium et que le Hib est ajouté sous forme non adjuvée ou adsorbé sur le phosphate d'aluminium.

19. Procédé selon la revendication 18, dans lequel la pertactine, le toxoïde de diphtérie, le toxoïde de pertussis, l'hémagglutinine filamenteuse, adsorbés sur l'hydroxyde d'aluminium, sont combinés avec les antigènes du virus de la polio inactivé, non adsorbés, avant d'être combinés aux antigènes adsorbés sur le phosphate d'aluminium.

20. Procédé selon l'une quelconque des revendications 12 à 15, dans lequel tous les antigènes du vaccin combiné sont adsorbés sur l'hydroxyde d'aluminium, avec la condition que le HBsAg est adsorbé sur le phosphate d'aluminium et que le Hib est ajouté sous forme non adjuvée ou adsorbé sur le phosphate d'aluminium.

21. Procédé selon la revendication 20, dans lequel les antigènes adsorbés sur l'hydroxyde d'aluminium sont combinés avant d'ajouter l'antigène HBsAg, adsorbé sur le phosphate d'aluminium.

22. Procédé selon les revendications 20 et 21, où du phosphate d'aluminium libre supplémentaire est ajouté au vaccin combiné avant addition extemporanée du Hib non adjuvé.

23. Procédé selon l'une quelconque des revendications 12 à 22, dans lequel un ou plusieurs des antigènes sélectionnés pour présaturer l'hydroxyde d'aluminium, ont été préalablement adosrbés sur phosphate d'aluminium.

24. Procédé selon la revendication 23, dans lequel le toxoïde de la diphtérie, adsorbé sur le phosphate d'aluminium, est un des antigènes sélectionnés pour présaturer l'hydroxyde d'aluminium.

25. Procédé selon l'une quelconque des revendications 1 à 24, dans lequel le vaccin combiné est tamponné avec la L-histidine.

26. Vaccin combiné comprenant :
a) DTPa, et
b) Hib adsorbé sur phosphate d'aluminium,
pouvant être obtenu par le procédé selon l'une quelconque des revendications 1 à 25.
